# EUROPEAN PATENT SPECIFICATION

(11) **EP 1 208 858 B1**
(45) Date of publication and mention of the grant of the patent: **14.06.2006**
(21) Application number: 01123287.3
(22) Date of filing: 05.10.2001
(51) Int. Cl.: A61M 5/20, A61M 5/32

(54) **Injector device for medical and cosmetic treatment**
Injektionsgerät zur medizinischen und kosmetischen Behandlung
Appareil d'injection pour le traitement médical et cosmétique

(30) Priority: 27.11.2000 IT MI002547
(43) Date of publication of application: 29.05.2002
(73) Proprietor: Versini, Paolo, 38068 Rovereto (Trento) (IT)
(72) Inventor: Versini, Paolo, 38068 Rovereto (Trento) (IT)
(74) Representative: Gislon, Gabriele

(56) References cited:
- WO-A-94/23777
- WO-A-95/29720
- US-A- 3 055 362
- US-A- 3 605 744
- US-A- 4 031 889
- US-A- 4 403 609
- US-A- 5 582 593

## Description

The present invention relates to a pneumatically operated injector device for medical and/or cosmetic treatment.

In particular, the invention relates to an injector device for mesotherapy, dermal stimulation and all medical therapies and cosmetic treatment requiring several injections to be performed in succession with a disposable syringe.

Known injection devices are composed of apparatuses designed to automatically perform injections and generally provided with a pistol frame to which an interchangeable syringe of the disposable type is fitted.

A device of this type is described for example in the European patent EP 0577538 in the name of Denance, relating to an injector device in which the front portion of the syringe engages with an element of the pistol able to guide the needle and predetermine its inclination during the injection; the rear portion of the syringe is engaged, by means of the head of the plunger, with a movable slide operated by an electric motor capable of transmitting the pressure required to inject the fluid to the plunger of the syringe. There is also another electric motor, again positioned inside the injector device that by means of the cams operates a supporting element of the syringe to make the needle of the syringe penetrate into and withdraw from the epidermis.

Injector devices of this type have some disadvantages. In the first place, there is a problem concerning manoeuvrability and weight of the device during the injection, due to the presence of at least one electric motor inside the device.

Moreover, the penetration speed of the needle is relatively low as it is linked to the limited capacities of the electric motor. It is known that the penetration speed of the needle is one of the characteristics responsible for the pain caused by the injection and, therefore, due to their limited speed, devices such as those described in the patent in the name of Denance do not help to reduce the pain caused by the penetration of the needle into the epidermis.

Another problem of this and other embodiments of the same inventor is linked to the use of a valve to prevent dripping interposed between the syringe and the needle. If, on the one hand, the presence of this valve solves the problems of dripping caused by inertia of the plunger of the syringe at the end of the injection, on the other it causes time to be wasted to replace the valve for reasons of hygiene at the end of each treatment and additional operations to adapt the disposable syringe to this device.

WO-A-95/29720, which represents the closest prior art discloses a device for injection of a medicament. The device comprises a housing having a medicament chamber, an injection needle communicating with the medicament chamber, a plunger in operable linkage to the needle. The housing is formed of two parts. The medicament chamber is accommodated along with releasable retaining means in the inferior part of the housing. The plunger is maintained in stored position by said releasable retaining means. When the plunger is activated, i.e. when a trigger bottom on the upper portion of the housing releases the retaining means, said upper portion is displaced towards the plunger and the air present in the headspace becomes compressed. The compressed air acts as a propulsive force to the plunger when the retaining means becomes released, thereby the needle penetrates into the tissue and subsequently, the medicament is injected. The needle returns into stored position by means of a spring inserted below the medicament chamber and by means of the residual compressed air above the plunger which serves to push back the upper portion of the housing from the corresponding lower portion.

US-A-4,403,609 relates to a needles vacuum compression injector comprising a cylindrical housing formed of two pieces threaded together in order to introduce a medicament insert into the inner space of the injector. A vacuum line and a compressed air line is used respectively for the immobilisation of the tissue and respectively for the injection of the medicament into the soft immobilised tissue.

In particularly, a vacuum annular passageway is formed within the cylindrical housing and it is joined to a vacuum line. When this vacuum line is opened, the tissue is drawn toward the forward end of the injector and the tissue is immobilised.

The medicament is forced to penetrate the soft immobilised tissue by means of driving the plunger plug of the medicament insert by a piston depressed by compressed air fed through compressed air line.

US-A-4,031,889 discloses a hypodermic syringe for automatically inserting a needle into the tissue, drawing a vacuum into said needle and injecting a medicament into said tissue. In the main housing of the syringe a compressed gas cartridge is accommodated. The gas cartridge has a membrane, which is ruptured when the cartridge is manually depressed. The compressed gas escapes from the cartridge and applies pressure to the cover of aspiration subassembly to propel injection assembly. The lower end of the hypodermic needle penetrates into the tissue. The control piston of the aspiration subassembly becomes displaced upwardly by the compressed gas and it creates a partial vacuum behind it, thereby displacing upwardly the piston of the injection assembly and effecting automatic aspiration. The compressed gas passes through perforations to the upper surface of the medicament piston and the increased gas pressure initiates injection of the medicament.

An attempt to implement injector devices with a high penetration speed of the needle into the epidermis is represented by the French patent FR 2594341 which envisages the use of a pneumatic jack to control an injection head provided with a needle connected to a piston syringe. Following penetration of the needle into the skin a reversible electric motor acts by means of a threaded screw on the piston of the syringe to determine the quantity of fluid to be injected.

A device of this type also has the problems of weight and manoeuvrability typical of electrically operated devices and, moreover, common disposable syringes cannot be used.

Also known are injector devices used in the veterinary field, such as the one described in the United States patent US4717384, in which the plunger of the syringe is operated pneumatically.

These devices for veterinary use also have disadvantages, as they do not offer the possibility to replace the syringe and do not have devices for automatic penetration of the needle into the epidermis.

The object of the present invention is to solve the aforesaid problems with an injection device capable of using disposable syringes, in which it is possible to mount the syringe on the injector device in a simple manner and without adjustments.

Another object of the present invention is to supply an injector device in which it is possible to adjust the speed of injection and penetration of the needle, that is reliable, manoeuvrable, light and simultaneously simple to be manufactured and inexpensive.

This object is attained with the present invention, which provides an injector device for medical and/or cosmetic treatment comprising a supporting element for a syringe provided with a plunger, the injector device comprising a hollow cylindrical element to house at least a part of the syringe and means to feed compressed gases to the hollow cylindrical element to operate the plunger of the syringe, and pneumatic means to alternately translate the syringe housing cylindrical element which is movable in relation to the supporting element, and in that said pneumatic means comprise a pneumatically operated cylinder and piston actuator and means to rigidly connect the syringe housing cylindrical element to the cylinder and piston actuator.

According to a preferred aspect of the invention, the cylindrical housing element comprises a pressing means interposed between the means to feed compressed gases and the plunger of the syringe to exert pressure on the plunger of the syringe.

According to another preferred aspect of the invention, the means to feed compressed gases to the cylindrical element comprise a cylindrical conduit coaxial to the rigid connection element between the cylindrical element and the piston of the cylinder and piston actuator, wherein the cylindrical conduit completely traverses the cylinder and piston actuator.

Thanks to the present invention it is possible to obtain an injector device that is manoeuvrable and light, with pneumatic actuation and hence high penetration speed of the needle into the epidermis, together with the possibility of using disposable syringes. Moreover, the possibility of varying the pressure allows an accurate control of the time and the quantity of fluid to be injected during the injection process.

Furthermore, the presence of a cylindrical element that encloses the syringe to conceal it completely offers psychological peace of mind to patients who are particularly sensitive or even phobic about the sight of this and, in particular, the needle.

The invention shall now be described, purely as a non-limiting example, with reference to the appended drawings, in which:
- fig. 1 shows the schematic and partially sectional side view of an embodiment of the injector device according to the invention at the start of the operation to inject the fluid;
- fig. 2 shows the schematic and partially sectional side view of an embodiment of the injector device according to the invention at the end of the operation to inject the fluid;
- fig. 3 shows the schematic and partially sectional side view of an embodiment of the injector device according to the invention at the end of the operation to withdraw the needle of the syringe from the epidermis;
- fig. 4 shows the schematic and partially sectional side view of an embodiment of the injector device according to the invention ready for use;
- fig. 5 shows a side view of a detail of the device in figure 1;
- fig. 6 shows a schematic view of the means to feed compressed gases according to the invention;
- fig. 7 shows a schematic side view of another embodiment of the injector device according to the invention.

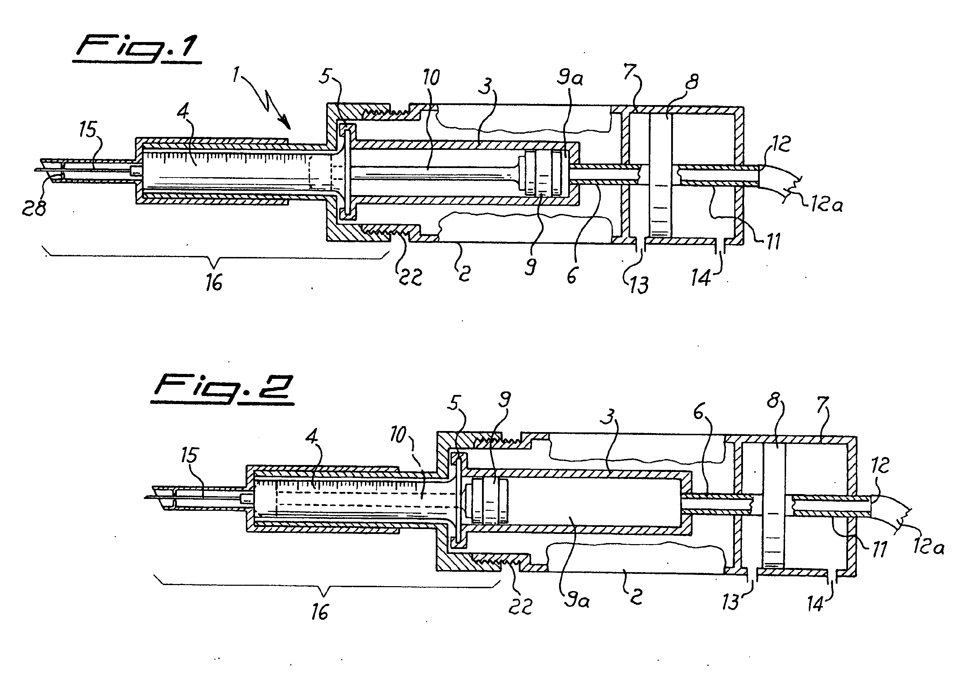

With reference to figures 1-4, an injector device for medical and/or cosmetic treatment, according to a specific aspect of the present invention, is indicated as a whole with the numerical reference 1.

The injector device has a tubular shaped element 2 to support a syringe 4, wherein a cylindrical element 3 is positioned which defines a housing for at least a part of the syringe 4. There are present also means 11, 12 to feed air or another compressed gas to the cylinder 3 to actuate, directly or indirectly, the plunger 10 of the syringe 4.

In particular, the syringe housing cylindrical element 3 has at one end fixing means 5, such as a bayonet coupling or clips, to hold and lock a common disposable syringe.

In this preferred embodiment (fig. 1) the body of the syringe projects completely from the cylinder 3, except for the flange of the syringe, engaged with the coupling 5 and the plunger 4 which is housed in the cylindrical element 3.

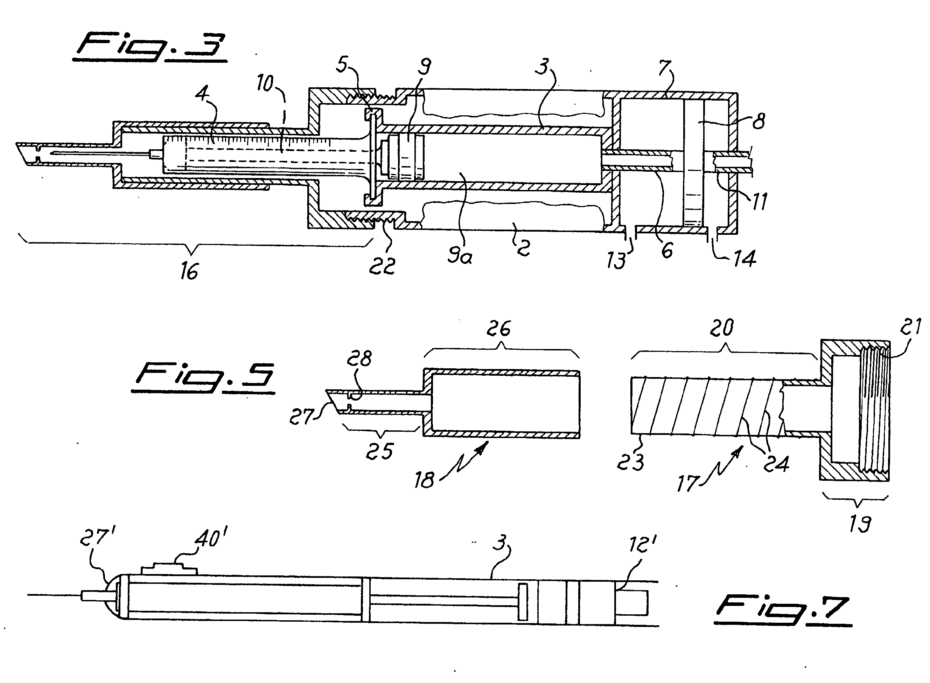

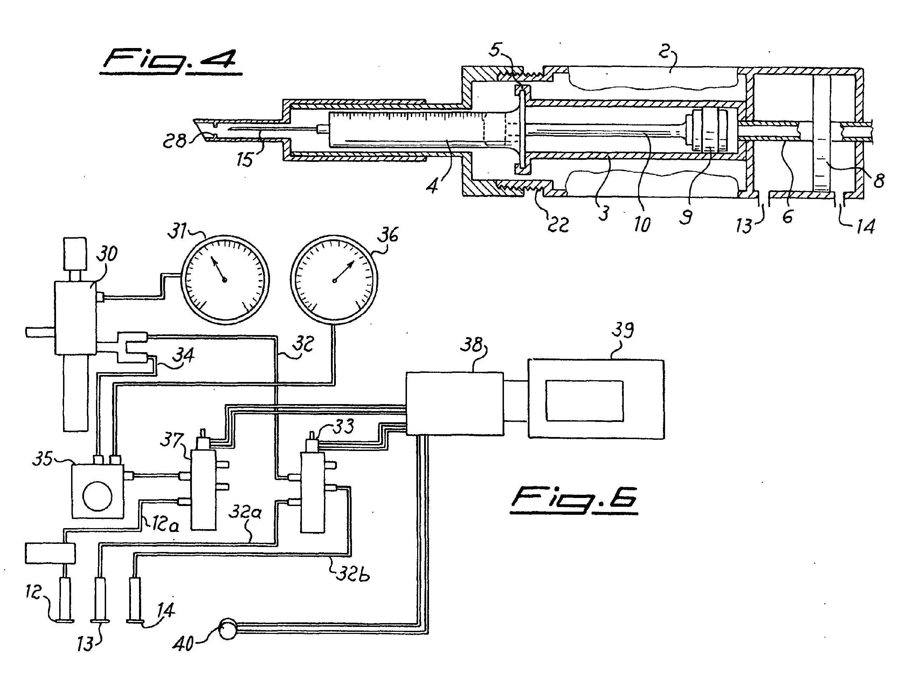

Preferably, the cylinder 3 is movable in relation to the support 2 to allow the needle of the syringe to be inserted into the skin or body of the patient. For this purpose pneumatic means are fitted to alternately translate the cylinder 3; these means comprise an actuator formed of a cylinder 7 and a piston 8 fitted inside the supporting element 2, and positioned upstream of the syringe housing cylinder 3 in relation to the flow of compressed gases. Fixed to the piston 8 is a rod 6 to which the housing cylinder 3 is connected rigidly; the movement of the piston 8 in the cylinder 7 is responsible for alternate translation of the syringe housing cylinder 3 inside the supporting element 2 and hence (fig. 1 and fig. 3), for the insertion and withdrawal of the syringe needle.

The rod 6 is preferably formed of a conduit and extends beyond the plunger 8 of the cylinder 7 with a portion 11 so as to completely traverse the cylinder 7, see figure 1, and have an aperture 12 positioned at one end of the supporting element 2. This aperture 12 is connected in a known manner (for example with the conduit 12a) to a source of air or similar compressed gases.

The cylinder 7 of the actuator has two apertures 13 and 14 for feeding and discharging the compressed gases, one positioned downstream and the other upstream of the piston 8 of the cylinder 7 in relation to the syringe.

The syringe housing element 3 has a pressing element 9 interposed to form a seal between the connection 6 of the cylinder rod 7 and the plunger of the syringe; said pressing element 9 is formed of a plunger that engages the plunger 10 of the syringe with pressure to move it towards the needle and empty the content of the syringe.

During use of the injector device 1, the feeding of the compressed gas from the aperture 14 located upstream of the plunger 8 forwardly translates the plunger 8 and, consequently, the syringe housing element 3, as can be seen in figure 1, effecting thereby the penetration of the syringe needle 15 into the epidermis. At the end of the fluid injection, caused by forward translation of the pressing element, see figure 2, the feeding of the compressed gas into the body of the piston cylinder through the aperture 13 positioned downstream of the plunger 8 causes the plunger 8 to backwardly translate, leading thereby to the discharge of the compressed gas, accumulated during the previous operation, from the aperture 14 positioned upstream of the plunger and, consequently, to the withdrawal of the needle 15 from the epidermis (see figure 3).

The end of the cylindrical support 2 downstream of the syringe housing element 3 also has a removable portion 16 that covers the needle 15 and the part of the syringe 4 projecting from the element 3. The removable portion 16 of the supporting element 2 allows, by means of its removal, the replacement of the syringe 4 when necessary or desired.

In detail, the removable portion 16 of the cylindrical supporting element 2 is formed of two hollow cylindrical portions 17, 18 with a variable section.

As can be seen in figure 5 the first 17 of said cylindrical portions is formed of a conduit composed of two cylindrical elements 19, 20 with different circular sections. The cylindrical element with the larger section 19 has at one end fixing means, such as a thread 21, to engage with a corresponding thread 22 positioned on the supporting element 2.

The external surface 23 of the cylindrical element with the smaller circular section 20 instead has ribbing 24 that allows it to connect selectively to the second cylindrical portion 18 of the removable portion 16 of the supporting element. This second cylindrical portion 18 is also formed of a conduit composed of two cylindrical elements 25, 26 with different circular sections. The cylindrical element with the larger section 25 has an appropriate dimension of the section to effect a selective connection to the second cylindrical portion 20 of the removable portion 16 of the supporting element 2.

In the present description "selective connection" is intended as a connection between the parts that allows gradual variation, through a successive series of balancing positions, of the total length of the removable portion 16 of the supporting element 2.

The cylindrical element with the smaller section 25 is designed to cover the needle and has at its free end an inclined aperture 27 from which the needle 15 of the syringe projects. The aperture 27 for projection of the needle is preferably inclined in relation to the direction of the needle by an angle of between 0° and 45°. During use of the injector device 16 the aperture 27 is positioned in contact with the skin of the patient and is responsible for the inclination of the injection.

Also fitted inside the second cylindrical portion 25 in the vicinity of the aperture 27are guiding means 28 for the needle. In greater detail, said guiding means 28 are represented by a circular ring that holds the syringe in its axis and acts as a safety device should the needle become detached from the syringe.

The fact that the overall length of the removable portion 16 of the supporting element 2 can be varied determines the maximum length of the needle projection from the aperture 27 and consequently the penetration depth of the needle into the epidermis.

The syringe housing cylindrical element 3 and the cylinder 7 and piston 8 actuator are connected to means to feed compressed gases. In particular, while the syringe housing cylindrical element 3 is connected to feed means through the conduit 11 that traverses the actuator, the latter is connected to said means through the apertures for feeding and discharging the compressed gases 13, 14 located upstream and downstream of the piston 8.

The means to feed compressed gases, as shown in figure 6, have three transport lines for compressed gases (two for the actuator and one for the syringe housing cylindrical element 3), a compressed gas source 30, such as a compressor or a tank and means to adjust the pressure and quantity of the compressed gases flowing through the transport lines.

In greater detail, the compressed gas source 30 provided with a gauge 31 to detect its internal pressure is connected through a first transport line 32 to a first solenoid valve 33 and through a second transport line 34 to a pressure reducer 35 that permits the pressure circulating in this line to be detected by means of a gauge 36 and to be kept constant.

The pressure reducer 35 is connected in turn to a second solenoid valve 37 and, by means of aperture 12 positioned at the end of the supporting element 2, with the chamber 9a defined by the pressing means 9 and by the syringe housing cylindrical element 3. This embodiment allows to obtain a chamber 9a with a restricted volume that guarantees high precision and repeatability of the travel of the pressing element 9 for the subsequent injections eventually required by the medical or cosmetic treatment.

The line 32 connecting the source 30 to the first solenoid valve 33 branches downstream of the solenoid valve 33 into two lines 32a, 32b that lead to the apertures 13, 14 for feeding and discharging the compressed gases of the piston cylinder 7.

The solenoid valves 33, 37 are controlled by an electronic card 38 such as a PLC used to programme the air feeding pressure and thus the penetration and withdrawal speed of the needle from the epidermis and the injection time.

The electronic card 38 is in turn connected to a display 39 to detect the functional parameters of the injector device 1 and to a push button panel used to programme the electronic card and operate the injector device. In particular, the button 40 to operate the device is positioned directly on the supporting element 2 that in a preferred embodiment has a pistol shaped frame.

To effect the injection the injector device 1 is positioned to be predisposed for use, as shown in figure 4, with the syringe housing element and the pressing means in the backwards position, on the area of skin involved in the medical or cosmetic treatment and, by pressing the start button 40, the cylinder 7 and piston 8 actuator is activated to act on the syringe housing cylindrical element 3 causing the penetration of the needle into the epidermis.

After the set pause time T1, the electronic card 40 commands the solenoid valve 37 to feed compressed gas into the chamber 9a of the syringe housing element 3. The compressed gas acts on the pressing element 9 for a pre-established time T2 to translate the plunger of the syringe to effect the fluid injection. After having been expelled a preset quantity of fluid dependent on the time T2 a second pause time T3 is effected to ensure that all the fluid required for the treatment has been delivered from the syringe to avoid any dripping during the subsequent operation of the needle withdrawal. After the time T3 the electronic card 40 commands the second solenoid valve 33 to move the piston cylinder 7 responsible for the withdrawal of the needle from the epidermis.

The pressure is regulated to allow variation of the penetration and withdrawal speed of the needle and the programming of the injection time T2 permits control of the quantity of fluid to be injected.

It is now possible, should the medical or cosmetic treatment require this, to effect another injection, following the same procedure, on the patient in an adjacent area of the epidermis.

At the end of the treatment, which may hence require several injections, and once the needle of the syringe has been withdrawn from the epidermis, the syringe may be replaced by removing the removable portion 16 of the supporting element 2.

If the medical or cosmetic treatment requires this, the injector device may also be operated manually. With this procedure the injection commences at the time in which pressure is exerted on the start button 40 and it continues for the entire time the button 40 remains pressed.

Thanks to the present invention it is possible to obtain an injector device 1 that is manoeuvrable, light, easy to manufacture and inexpensive, that permits regulation of the quantity of fluid to be injected and capable of solving the problems correlated to the penetration speed of the needle according to prior art. Furthermore, the presence of a cylindrical element that encloses the syringe concealing it completely offers psychological peace of mind to patients who are particularly sensitive or even phobic about the sight of the needle.

The above described embodiment of the invention may undergo numerous modifications and variants all falling within the scope of its protection. For example, according to an alternative embodiment, shown in figure 7, a hermetically closed hollow cylindrical element 3 may be provided to house the syringe, which simultaneously acts as a supporting element. At one end of said element 3 is an aperture for the needle 27' and at the other end an aperture 12' for connection to means to feed the compressed gas.

An operating button 40' positioned on the syringe housing cylindrical element 3 determines the actuation of the means to feed compressed gases, which in this case will only supply the transport line for the piston of the syringe.

## Claims

1. Injector device for medical and/or cosmetic treatment comprising
◆ a supporting element (2) for a syringe (4) provided with a plunger (10),
◆ a cylindrical element (3) to house at least a part of said syringe (4);
◆ means to feed compressed gases (11, 12) to said cylindrical element (3) to operate said plunger of the syringe,
**characterized in that** further comprising
◆ pneumatic means to alternately translate said syringe housing cylindrical element (3) which is movable in relation to the supporting element (2), and **in that**
said pneumatic means comprises a pneumatically operated cylinder (7) and piston (8) actuator and means to rigidly connect (6) said syringe housing element (3) to the piston (8) of said actuator.

2. Injector device according to claim 1, **characterized in that** said cylinder (7) has two apertures (13, 14) for feeding and discharging the compressed gases, one positioned downstream and the other upstream of the piston (8) of the cylinder (7) in relation to the syringe.

3. Injector device as claimed in claim 1 or 2, **characterized in that** said cylindrical housing element (3) comprises a pressing means (9) interposed between the means to feed compressed gases (11, 12) and the plunger of the syringe to exert pressure on said plunger (10) of the syringe (4).

4. Injector device as claimed in claims from 1 to 3, **characterized in that** the means to feed compressed gases (11, 12) to the cylindrical element comprise a cylindrical conduit coaxial to the rigid connection element (6) between said cylindrical element (3) and the piston of the cylinder and piston actuator (8), said conduit completely traversing said cylinder (3) and piston actuator (8).

5. Injector device as claimed in any of the previous claims **characterized in that** said supporting element (2) is a hollow cylindrical element that encloses said syringe housing cylindrical element (3), said cylinder and piston actuator, said means to feed compressed gases and a removable portion to cover the syringe.

6. Injector device as claimed in claim 5, **characterized in that** said removable portion (16) comprises two hollow cylindrical portions (17, 18) with variable sections that can be selectively connected to adjust the total length of said removable portion.

7. Injector device as claimed in claim 1, **characterized in that** said syringe housing cylindrical portion is the supporting element and comprises an aperture for the needle of the syringe (15) positioned at one end of the cylindrical element (3) and the means to feed compressed gases (11, 12) positioned at the other end of the cylindrical element.

8. Injector device as claimed in any of the previous claims **characterized in that** said means to feed compressed gases (11, 12) to the cylindrical housing element and to the cylinder (7) and piston actuator (8) comprise sources (30) to feed compressed gases, transport lines for compressed gases and apparatus to control the pressure and the quantity of compressed gas circulating in said transport lines.

9. Injector device as claimed in claim 8, **characterized in that** it has three transport lines: two for feeding and discharging the compressed gases (32a, 32b) from the actuator and one for feeding the compressed gases (12a) to the syringe housing cylindrical element.

10. Injector device as claimed in claim 8 or 9, **characterized in that** said apparatuses to control the quantity and the pressure circulating in the transport lines for compressed gases (12a, 32a, 32b) comprise at least one valve, at least one electronic card (38), at least one element to detect data (36) and at least one push-button panel (40).

## Patentansprüche

1. Injektionsgerät zur medizinischen und/oder kosmetischen Behandlung, umfassend
• ein Trägerelement (2) für eine Spritze (4), die mit einem Kolben (10) versehen ist;
• ein zylindrisches Element (3) zur Unterbringung wenigstens eines Teils der Spritze (4);
• Hilfsmittel für die Zuleitung komprimierter Gase (11, 12) zum zylindrischen Element (3) zur Betätigung des Kolbens der Spritze;
**dadurch gekennzeichnet, dass** es des Weiteren
• ein pneumatisches Hilfsmittel zur alternierenden Verschiebung des zylindrischen Spritzengehäuseelements (3) umfasst, das bezüglich des Trägerelements (2) beweglich ist, und dass
das pneumatische Hilfsmittel ein pneumatisch betriebenes Betätigungselement aus Zylinder (7) und Druckkolben (8) und Hilfsmittel zur starren Verbindung (6) des Spritzengehäuseelements (3) mit dem Druckkolben (8) des Betätigungselements umfasst.

2. Injektionsgerät nach Anspruch 1, **dadurch gekennzeichnet, dass** der Zylinder (7) zwei Öffnungen (13, 14) für die Zuleitung und Ableitung der komprimierten Gase aufweist, wobei die eine nach und die andere vor dem Druckkolben (8) des Zylinders (7) in Verbindung mit der Spritze angeordnet ist.

3. Injektionsgerät nach Anspruch 1 oder 2, **dadurch gekennzeichnet, dass** das zylindrische Gehäuseelement (3) ein Druckhilfsmittel (9) umfasst, das zwischen den Hilfsmitteln für die Zuleitung der komprimierten Gase (11, 12) und dem Kolben der Spritze angeordnet ist, um Druck auf den Kolben (10) der Spritze (4) auszuüben.

4. Injektionsgerät nach den Ansprüchen 1 bis 3, **dadurch gekennzeichnet, dass** die Hilfsmittel für die Zuleitung der komprimierten Gase (11, 12) zum zylindrischen Element einen zylindrischen Kanal umfassen, der coaxial zu dem starren Verbindungselement (6) zwischen dem zylindrischen Element (3) und dem Druckkolben des Betätigungselements aus Zylinder (7) und Druckkolben (8) ist, wobei der Kanal das Betätigungselement aus Zylinder (7) und Druckkolben (8) vollständig durchquert.

5. Injektionsgerät nach einem der vorstehenden Ansprüche, **dadurch gekennzeichnet, dass** das Trägerelement (2) ein zylindrisches Hohlelement ist, das das zylindrische Spritzengehäuseelement (3), das Betätigungselement aus Zylinder und Druckkolben, die Hilfsmittel für die Zuleitung der komprimierten Gase und einen abnehmbaren Teil zur Abdeckung der Spritze umschließt.

6. Injektionsgerät nach Anspruch 5, **dadurch gekennzeichnet, dass** der abnehmbare Teil (16) zwei hohle zylindrische Teile (17, 18) mit variablen Abschnitten umfasst, die selektiv angeschlossen werden können, um die Gesamtlänge des abnehmbaren Teils einzustellen.

7. Injektionsgerät nach Anspruch 1, **dadurch gekennzeichnet, dass** der zylindrische Spritzengehäuseteil das Trägerelement ist und eine Öffnung für die Nadel der Spritze (15) umfasst, die an einem Ende des zylindrischen Elements (3) angeordnet ist, und die Hilfsmittel für die Zuleitung der komprimierten Gase (11, 12) am anderen Ende des zylindrischen Elements angeordnet sind.

8. Injektionsgerät nach einem der vorstehenden Ansprüche, **dadurch gekennzeichnet, dass** die Hilfsmittel für die Zuleitung der komprimierten Gase (11, 12) zum zylindrischen Gehäuseelement und zum Betätigungselement aus Zylinder (7) und Druckkolben (8) Quellen (30) für die Zuleitung komprimierter Gase, Transportleitungen für die komprimierten Gase und Vorrichtungen zur Steuerung des Drucks und der Menge der in den Transportleitungen zirkulierenden komprimierten Gase umfassen.

9. Injektionsgerät nach Anspruch 8, **dadurch gekennzeichnet, dass** es drei Transportleitungen aufweist: zwei für die Zuleitung und Ableitung der komprimierten Gase (32a, 32b) aus dem Betätigungselement und eine für die Zuleitung der komprimierten Gase (12a) zum zylindrischen Spritzengehäuseelement.

10. lnjektionsgerät nach Anspruch 8 oder 9, **dadurch gekennzeichnet, dass** die Vorrichtungen zur Steuerung der Menge und des Drucks, die in den Transportleitungen für die komprimierten Gase (12a, 32a, 32b) zirkulieren, wenigstens ein Ventil, wenigstens eine elektronische Karte (38), wenigstens ein Element zur Erfassung von Daten (36) und wenigstens ein Drucktastenfeld (40) umfassen.

## Revendications

1. Appareil d'injection pour le traitement médical et/ou cosmétique comprenant :
un élément de support (2) pour une seringue (4) munie d'un piston plongeur (10) ;
un élément cylindrique (3) destiné à loger au moins une partie de ladite seringue (4) ;
un moyen (11, 12) destiné à alimenter en gaz comprimés ledit élément cylindrique (3) pour faire fonctionner ledit piston plongeur de la seringue,
**caractérisé en ce qu'**il comprend en outre :
un moyen pneumatique pour translater alternativement ledit élément cylindrique de logement de la seringue lequel est mobile par rapport à l'élément de support (2), et **en ce que**
ledit élément pneumatique comprend un cylindre (7) à fonctionnement pneumatique et un actionneur de piston (8) et un moyen (6) pour relier de façon rigide ledit élément (3) de logement de la seringue au piston (8) dudit actionneur.

2. Appareil d'injection selon la revendication 1, **caractérisé en ce que** ledit cylindre (7) présente deux ouvertures (13, 14) destinées à l'entrée et la sortie des gaz comprimés, l'une étant disposée en aval et l'autre en amont du piston (8) du cylindre (7) par rapport à la seringue.

3. Appareil d'injection selon la revendication 1 ou 2, **caractérisé en ce que** ledit élément cylindrique de logement (3) comprend un moyen de pression (9) interposé entre le moyen d'alimentation en gaz comprimés (11, 12) et le piston plongeur de la seringue de façon à exercer une pression sur ledit piston plongeur (10) de la seringue (4).

4. Appareil d'injection selon les revendications 1 à 3, **caractérisé en ce que** ledit moyen d'alimentation en gaz comprimé (11, 12) de l'élément cylindrique comprend un conduit cylindrique coaxial avec l'élément de liaison rigide (6) entre ledit élément cylindrique (3) et le piston du cylindre et avec l'actionneur de piston (8), ledit conduit traversant en leur totalité lesdits cylindre (3) et actionneur de piston (8).

5. Appareil d'injection selon l'une quelconque des revendications précédentes, **caractérisé en ce que** ledit élément de support (2) est un élément cylindrique creux qui inclut ledit élément cylindrique (3) de logement de la seringue, lesdits cylindre et actionneur à piston, ledit moyen d'alimentation en gaz comprimés et une partie amovible pour recouvrir la seringue.

6. Appareil d'injection selon la revendication 5, **caractérisé en ce que** ladite partie amovible (16) comprend deux parties cylindriques creuses (17, 18) de sections variables qui peuvent être sélectivement connectées de façon à régler la longueur totale de ladite partie amovible.

7. Appareil d'injection selon la revendication 1, **caractérisé en ce que** ladite partie cylindrique de logement de la seringue constitue l'élément de support et comprend une ouverture pour l'aiguille (15) de la seringue positionnée à une extrémité de l'élément cylindrique (3) et pour le moyen d'alimentation en gaz comprimés (11, 12) positionné à l'autre extrémité de l'élément cylindrique.

8. Appareil d'injection selon l'une quelconque des revendications précédentes, **caractérisé en ce que** ledit moyen d'alimentation en gaz comprimés (11, 12) vers l'élément cylindrique de logement et vers le cylindre (7) et l'actionneur de piston (8) comprend des sources (30) d'alimentation en gaz comprimés, des canalisations de transport pour les gaz comprimés et un dispositif pour contrôler la pression et la quantité de gaz comprimé circulant dans lesdites canalisations.

9. Appareil d'injection selon la revendication 8, **caractérisé en ce qu'**il comprend trois canalisations de transport : deux (32a, 32b) pour l'alimentation et l'évacuation des gaz comprimés pour l'actionneur et une (12a) pour l'alimentation en gaz comprimés de l'élément cylindrique de logement de la seringue.

10. Appareil d'injection selon la revendication 8 ou 9, **caractérisé en ce que** lesdits dispositifs de contrôle de la quantité et de la pression circulant dans les canalisations de transport des gaz comprimés (12a, 32a, 32b) comprennent au moins une soupape, au moins une carte électronique (38), au moins un élément de détection de données (36) et au moins un bouton de commande (40).
